# EUROPEAN PATENT SPECIFICATION

(11) **EP 2 068 770 B1**
(45) Date of publication and mention of the grant of the patent: **23.02.2011**
(21) Application number: 07871051.4
(22) Date of filing: 29.08.2007
(51) Int. Cl.: A61F 2/24

(54) **SADDLE-SHAPED ANNULOPLASTY RING**
SATTELFÖRMIGER ANNULOPLASTIERING
ANNEAU D'ANNULOPLASTIE EN FORME DE SELLE

(30) Priority: 01.09.2006 US 514624
(43) Date of publication of application: 17.06.2009
(73) Proprietor: Edwards Lifesciences Corporation, Irvine, CA 92614 (US)
(72) Inventor: INGLE, Aaron, West Linn, OR 97068 (US); ADZICH, Vaso, Santa Ana, CA 92705 (US)
(74) Representative: Eisenführ, Speiser & Partner
(86) International application number: PCT/US2007/077133
(87) International publication number: WO 2008/070232

(56) References cited:
- WO-A-90/09153
- WO-A-03/041617
- WO-A-2007/050506
- US-A1- 2005 131 533
- US-A1- 2006 129 236

## Description

### Field of the Invention

The present invention refers to a prosthetic annuloplasty ring for a mitral valve, in particular for correcting pathologies associated with mitral valve prolapse, for example, Barlow's syndrome or myxomatous disease.

### Background of the Invention

In the operation of the heart, returning blood enters the right atrium and passes through the tricuspid valve into the right ventricle. From there, blood is pumped through the pulmonary valve and the pulmonary artery to the lungs. Oxygenated blood enters the left atrium and passes into the left ventricle through the mitral valve. Healthy mitral valve leaflets "coapt" or meet near the middle of the blood flow path and are attached to papillary muscles within the interior of the left ventricle by a number of stringy chordae tendinae. During systole, the mitral valve closes and the aortic valve opens, thus preventing blood from regurgitating into the left atrium and forcing blood into the aorta, and from there throughout the body. Because of the high pressures associated with the left ventricle during systole, proper mitral valve function to prevent back flow through the system is extremely important.

Mitral regurgitation is one of the most common valvular malfunctions in the adult population. Mitral valve prolapse is the most common cause of mitral regurgitation in North America and is believed to affect at least 5 to 10 percent of the population in the U.S. Women are affected about twice as often as men. Mitral valve prolapse has been diagnosed as Barlow's syndrome, billowing or balloon mitral valve, floppy mitral valve, floppy-valve syndrome, myxomatous mitral valve, prolapsing mitral leaflet syndrome, or systolic click-murmur syndrome. Some forms of mitral valve prolapse seem to be hereditary, though the condition has been associated with Marfan's syndrome, Grave's disease, and other disorders.

Barlow's disease is characterized by myxoid degeneration and appears early in life, often before the age of fifty. Patients topically present with a long history of systolic murmur and may experience valve infection, arrhythmias and atypical chest pain. Some cases are asymptomatic, but a pronounced midsystolic click with or without late systolic murmur, usually indicates the presence of this disorder. South African cardiologist John B. Barlow was the first to interpret this auscultation syndrome, known for decades as an expression of a mitral valve prolapse. In Barlow's disease, one or both leaflets of the mitral valve protrude into the left atrium during the systolic phase of ventricular contraction. The valve leaflets are thick with considerable excess tissue, producing an undulating pattern at the free edges of the leaflets. The chordae are thickened, elongated and may be ruptured. Papillary muscles are also occasionally elongated. The annulus is dilated and sometimes calcified. Of course, some of these symptoms present in other pathologies, and therefore the present application will refer to mitral valve prolapse as a catch-all for the various diagnoses, including Barlow's syndrome.

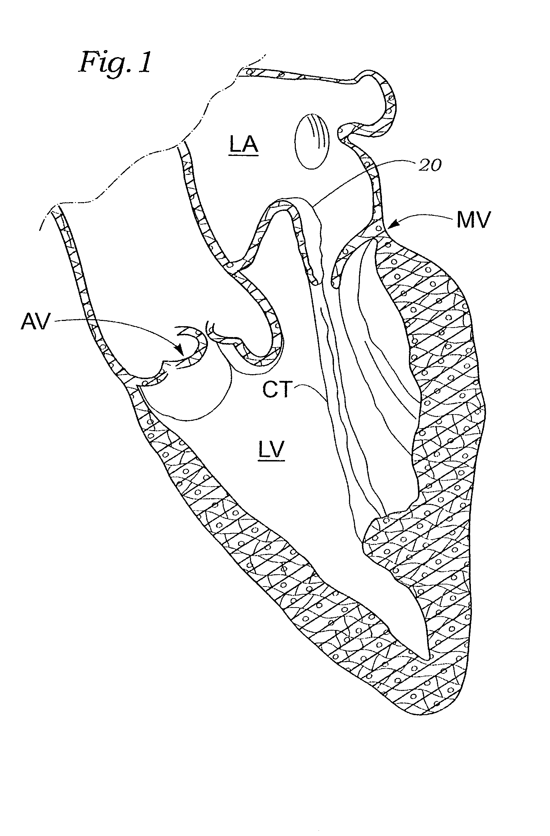

Fig. 1 is an enlarged view of the left ventricle LV illustrating mitral valve prolapse, such as seen with Barlow's syndrome. The anterior leaflet 20 of the mitral valve MV is shown thickened and lengthened from its normal configuration. As a result, the leaflet 20 is shown flopping upward into the left atrium LA. This excess tissue, or redundancy, often prevents the anterior and posterior leaflets from properly coapting, resulting in mitral regurgitation.

In patients with degenerative mitral valve disease, valve repairs using mitral valvuloplasty valve reconstruction, or annuloplasty have been the standards for surgical correction of mitral regurgitation and have provided good long-term result. A rigid support ring (e.g., Carpentier-Edwards Classic®), a semi-flexible ring (e.g., Carpentier-Edwards Physio®), or a flexible ring (e.g., Cosgrove-Edwards®) may be used. Other repair techniques include: quadrangular resection of the prolapsing portion of the posterior leaflet; transposition of a portion of the posterior leaflet to the anterior leaflet to correct anterior-leaflet prolapse; commissurotomy combined with ring annuloplasty; replacement of a chordae tendinae with sutures; and plication (or resection) of the anterior leaflet. A commonly used repair is the so-called "sliding technique" introduced by Dr. Alain Carpentier, which involves quadrangular resection followed by cutting the posterior leaflet and reconstruction to shorten this leaflet. Also, in the early 1990's, Dr. Ottavio Alfieri introduced the concept of edge-to-edge heart valve repair. This repair technique consists of suturing the edges of the leaflets at the site of regurgitation, either at the paracommissural area (e.g.: A1-P1 segments: para commissural repair) or at the middle of the valve (e.g.: A2-P2 segments: double orifice repair). Each of these aims to tighten up the mitral annulus to a more normal shape and restore coaptation.

The advantages of repair over replacement have been widely demonstrated; however, studies have shown that mitral valve repair is performed in less than half of surgical procedures involving the mitral valve, and even fewer repairs are performed in patients with complex mitral regurgitation (e.g., Barlow's disease, bileaflet prolapse and annular calcification). Despite adequate tissue resection and placement of an annuloplasty ring or band, patients may have residual mitral regurgitation associated with systolic anterior motion (SAM) of the anterior leaflet. SAM occurs when the elongated leaflet is pulled into the left ventricular outflow tract (LVOT). This leads to partial LVOT obstruction and hemodynamic instability. This scenario is not an uncommon incident following an otherwise successful mitral valve repair and can be very difficult to treat with existing repair techniques and devices, and may require mitral valve replacement rather than the preferred of valve repair.

Despite accepted treatments for correcting mitral valve prolapse, for example Barlow's syndrome, there is a need for a simpler and more effective approach that takes into account various patient geometries.

### Summary of the Invention

The present invention provides, in one aspect, a mitral annuloplasty ring comprising a closed and generally rigid ring body arranged around a flow axis having an upward direction and a downward direction. The downward direction corresponds to the direction of blood flow through the mitral valve annulus when the annuloplasty ring is implanted. The ring body has at least two lowermost points on opposite sides of the ring body that lie in a reference plane generally perpendicular to the flow axis. The ring body exhibits, in top plan view along the flow axis, a rounded isosceles triangular configuration with a long relatively straight anterior segment substantially extending between two trigones and intended to be implanted against the anterior aspect of the mitral annulus. Two shorter relatively straight posterior segments join at a convex posterior apex and together define a posterior portion of the ring body. The posterior portion is intended to be implanted against the posterior aspect of the mitral annulus. In elevational view parallel to the reference plane, each of the anterior segment and the posterior portion has an upward bow therein relative to the two lowermost points of the ring, wherein the anterior segment bows upward farther from the reference plane than the posterior portion.

In accordance with a preferred embodiment, the ring body defines a minor axis extending between and bisecting the anterior segment and posterior portion and a major axis extending perpendicularly thereto, the major and minor axes being generally perpendicular to the flow axis and each having dimensions across the ring body. Desirably, the posterior portion of the ring body has an outward bulge more pronounced than adjacent sections. The upward bow in the posterior portion may have an angular extent approximately equal to the outward bulge.

Preferably, the magnitude of the posterior upward bow is between about 2.9-4.5 mm, and the magnitude of the anterior upward bow is between about 4-7 mm, and at least 1 mm greater than the posterior bow. Stated in relative terms, the relative magnitude of the posterior upward bow is between about 9%-13% of the major axis dimension. At the same time, the relative magnitude of thy anterior upward bow may be between about 14%-20% of the major axis dimension.

Another aspect of the invention pertains to a mitral annuloplasty ring comprising a closed and generally rigid ring body arranged around a flow axis having an upward direction and a downward direction. The downward direction corresponds to the direction of blood flow through the mitral valve annulus when the annuloplasty ring is implanted. The ring body has at least two lowermost points on opposite sides of the ring body that lie in a reference plane generally perpendicular to the flow axis. In top plan view along the flow axis, a relatively straight anterior segment substantially extends between two trigones and is intended to be implanted against the anterior aspect of the mitral annulus. A generally convex posterior portion of the ring body extends between two trigones opposite the anterior segment and is intended to be implanted against the posterior aspect of the mitral annulus. A minor axis extends between the anterior segment and an apex of the posterior portion, and a major axis extends perpendicularly thereto across the widest part of the ring body. The major and minor axes are generally perpendicular to the flow axis and each have dimensions across the ring body. The posterior portion of the ring body has an outward bulge that creates a minor axis to major axis dimension ratio of between about 3.3:4 (82.5%) and 4:4 (100%). In elevational view parallel to the reference plane, each of the anterior segment and the posterior portion has an upward bow therein relative to the two lowermost points of the ring, wherein the anterior segment bows upward farther from the reference plane than the posterior portion.

The ring body may have, in top plan view along the flow axis, a rounded isosceles triangular configuration defined by the anterior segment and two shorter relatively straight posterior segments joined at a convex posterior apex and together defining the posterior portion. Preferably, the magnitude of the posterior upward bow is between about 2.9-4.5 mm, and the magnitude of the anterior upward how is between about 4-7 mm. Stated in relative terms, the relative magnitude of the posterior upward bow is between about 9%-13% of the major axis dimension, while the relative magnitude of the anterior upward bow may be between about 14%-20% of the major axis dimension.

In another embodiment of the invention, a mitral annuloplasty ring includes a closed and generally rigid ring body arranged around a flow axis having an upward direction and a downward direction. The downward direction corresponds to the direction of blood flow through the mitral valve annulus when the annuloplasty ring is implanted. The ring body exhibits, in top plan view along the flow axis, a relatively straight anterior segment substantially extending between two trigones and intended to be implanted against the anterior aspect of the mitral annulus. Two posterior segments join at a convex posterior apex and together define a posterior portion of the ring body intended to be implanted against the posterior aspect of the mitral annulus. In elevational view parallel to the reference plane, each of the anterior segment and the posterior portion has an upward bow therein relative to the two lowermost points of the ring, wherein the anterior segment bows upward farther from the reference plane than the posterior portion. In a preferred form, the ring body defines lowermost flat segments on opposite sides of the posterior portion in which are located the two lowermost points of the ring. The upward bow in the anterior segment is desirably between 1-2 mm higher than the upward bow in the posterior portion. Preferably, the magnitude of the posterior upward bow is between about 2.9-4.5 mm, and the magnitude of the anterior upward bow is between about 4-7 mm.

### Brief Description of the Drawings

Features and advantages of the present invention will become appreciated as the same become better understood with reference to the specification, claims, and appended drawings wherein:

Fig. 1 is an enlarged sectional view of the left ventricle of a human heart illustrating one configuration of distended mitral valve leaflets seen with mitral valve prolapse;

Figs. 2-6 are various views of a mitral annuloplasty ring of the present invention having both anterior and posterior upward bows;

Figs. 7-10 are various views of an exemplary mitral annuloplasty ring body forming the primary structural component of the annuloplasty ring of the present invention, some indicating a number of dimensional parameters;

Figs. 11 and 12 are perspective views of a mitral annuloplasty ring of the present invention being lowered onto an abnormal mitral valve as viewed from the posterior aspect and from one side thereof; and

Figs. 13 and 14 are perspective views of a mitral annuloplasty ring of the present invention after having been anchored to an abnormal mitral valve as viewed from the posterior aspect and from one side thereof.

### Detailed Description of the Preferred Embodiments

The present invention provides a novel annuloplasty ring for correcting pathologies associated with mitral valve prolapse, also known by a number of other names given above, including Barlow's syndrome. With this pathology, the mitral valve leaflets are distended (i.e., stretched, lengthened, swelled, thickened) or in general have become loose and floppy such that they do not properly coapt. In contrast to prior repair techniques, the annuloplasty ring of the present invention reduces or eliminates the need for a sliding annuloplasty. Furthermore, instead of attempting to constrict the mitral annuls by the addition of an annuloplasty ring that is under-sized with respect to the existing annulus, the present invention accommodates the excess material of the leaflets by providing a larger support ring than has previously been utilized. Typical annuloplasty support rings have a long or major dimension and a short or minor dimension, with the conventional ratio of the minor to major dimension being at most 3:4 (75%), and typically less. The present invention provides an annuloplasty ring that has a significantly increased minor to major dimension ratio of between about 3.3:4 (82.5%) and 4:4 (100%).

Annuloplasty rings of the present invention are desirably made of material(s) that are "generally rigid" and will initially resist distortion when subjected to the stress imparted thereon by the mitral valve annulus of an operating human heart. In this sense, "distortion" means substantial permanent deformation from a predetermined or manufactured shape; the opposite concept of which is "elastic" meaning the ability to recover the ring shape in the absence of an external force. A number of "generally rigid" materials can be utilized that will perform this function, including various bio-compatible polymers and metals and/or alloys. Certain polyesters that resist distortion and also rapid degradation within the body may be used (a material that degrades slowly may provide the required initial support). In a preferred embodiment, at least an inner core or body of the annuloplasty ring of the present invention is made of a suitable metal, such as titanium or its alloys, or ELGILOY made by Elgiloy, L.P. of Elgin, III., U.S.A. The core or ring body may be one piece, or may include a plurality of concentric or otherwise cooperating elements. The addition of a silicone tube or band around the ring body and a suture-permeable fabric on the exterior of the ring are also desirably to provide purchase for anchoring sutures.

In a preferred embodiment, the annuloplasty ring of the present invention comprises a continuous ring body made of a titanium alloy. A soft tubular sleeve or outer band, which may be formed from silicone, surrounds the ring body and helps conform tissue to the ring after implantation. Finally, a tubular fabric covering around the silicone sleeve provides an anchoring platform for sutures or other attachment devices such as staples. The fabric covering is typically Dacron (polyethylene terephthalate). The tubular fabric covering around the silicone sleeve provide an interface for securing the annuloplasty ring to the mitral annulus, although other interfaces are contemplated. For example, rings having outward hooks or barbs are known in the art.

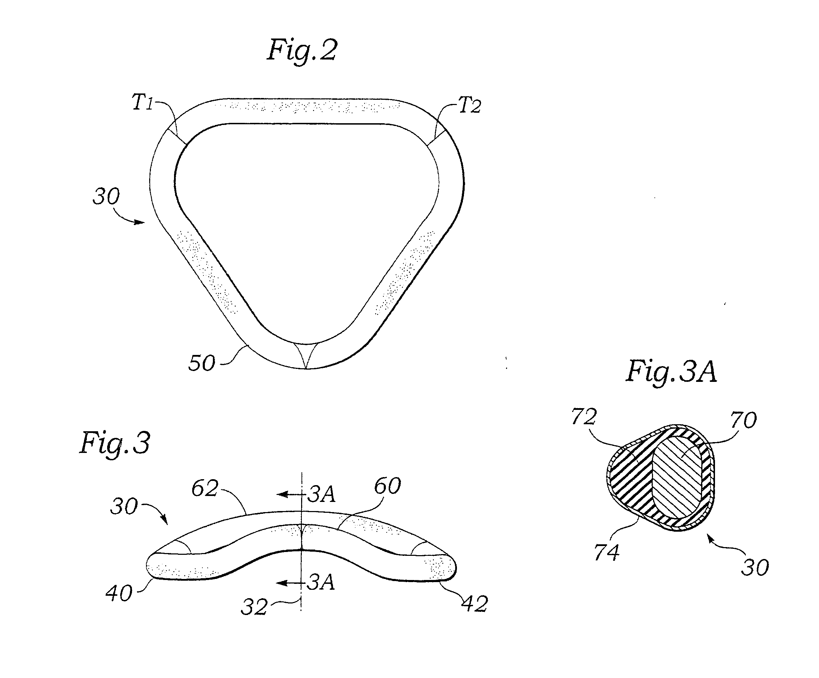

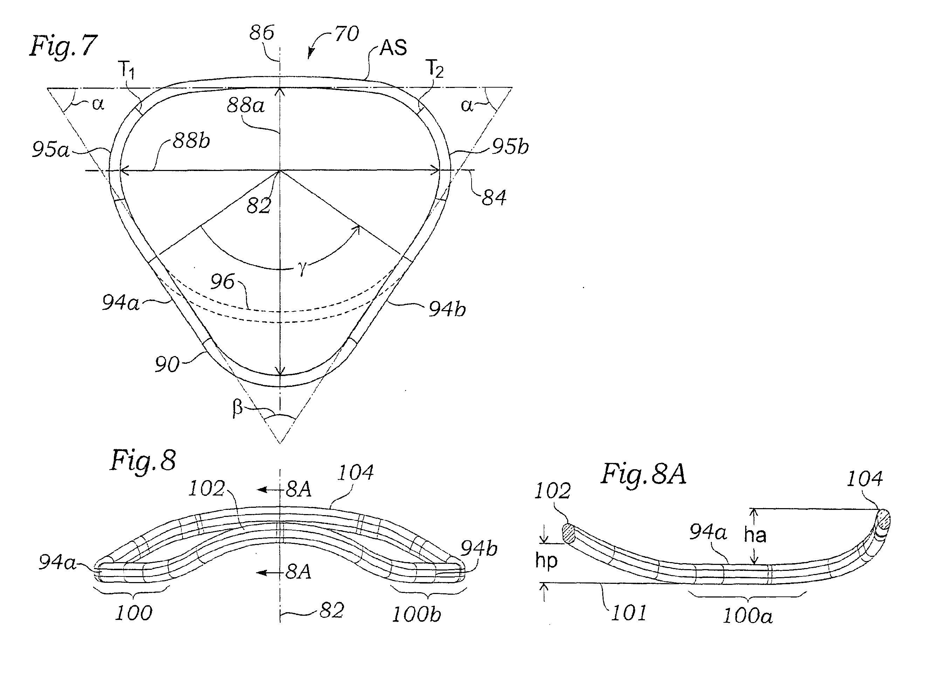

With reference now to Figs. 2-6, an exemplary mitral annuloplasty ring 30 is shown in various plan, elevational and sectional views. These views illustrate the completed ring so that a fabric covering is all that is visible. An exemplary ring body will be described below with respect to Figs. 7-10, though it should be understood that the shape of the completed ring follows closely the shape of the inner ring body which provides its primary structural support.

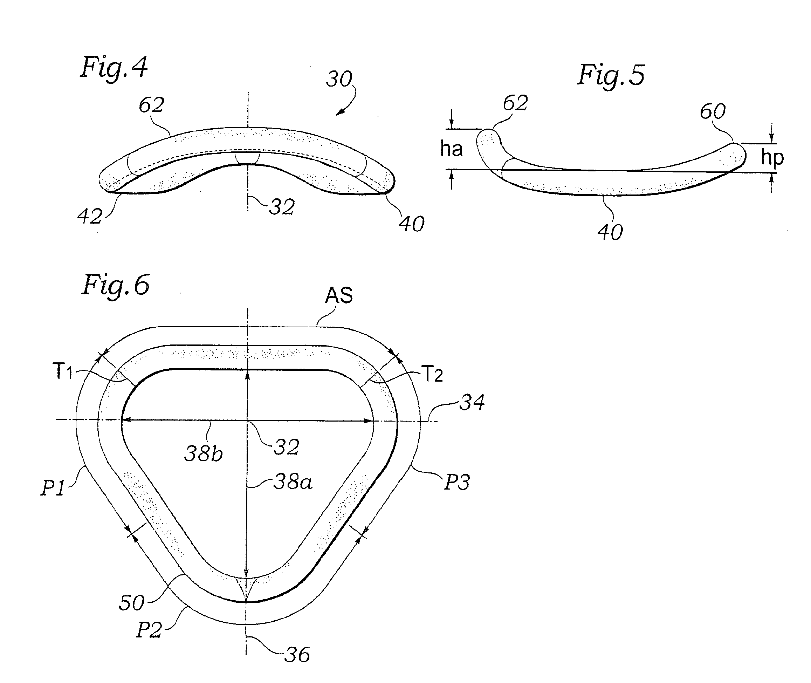

As seen in Figs. 2 and 6, the annuloplasty ring 30 has a rounded isosceles triangular shape (closed) in plan view and is oriented about a central flow axis 32, but is somewhat saddle-shaped when all three dimensions are considered. The flow axis 32 defines an upward direction and a downward direction, corresponding to the top and bottom of the page relative to the ring 30, as seen in Figs. 3 and 4. The downward direction corresponds to the direction of blood flow through the mitral valve annulus from the left atrium to the left ventricle, such that up is synonymous with the inflow direction and down with the outflow direction of the valve.

Looking along the flow axis 32 in Fig. 6, the ring 30 has a major axis 34 perpendicular to a minor axis 36, the major and minor axes being perpendicular to the flow axis. It should also be understood that the "flow axis" here may not necessarily be the center of the volumetric flow through the annulus, but is instead orthogonal to the major and minor axes 34, 36, and therefore defines the gross direction of flow. A minor axis dimension 38a is shown extending across the anterior of the ring 30 in plan view. Likewise, a major axis dimension 38b is shown extending horizontally across the interior of the ring. Desirably, the ratio of the minor axis dimension 38a to the major axis dimension 38b is about 3.5:4 (87.5%).

Before continuing, it is important to understand the reference directions associated with the annuloplasty ring 30. First of all, the concept of a central axis is somewhat of a misnomer as the plan view of the ring, such as in Fig. 6, shows that the ring is not axi- or tri-symmetric, or otherwise possessing of symmetry such that a center can be identified. Instead, the "central" or flow axis 32 passes through the intersection of the major axis 34 and minor axis 36 by convention. More significant is the direction of the flow axis 32, or stated another way the orientation of the reference plane defined by the intersection of the major and minor axes 34, 36. For purpose of discussion, the reader will understand that the mitral annulus of a normal, healthy heart lies generally in a plane defined perpendicular to the average blood flow direction through the mitral valve. This plane is often used to describe the orientation of the mitral annulus within the heart, wherein the upward direction in Figs. 3 and 4 generally coincides with upward when the patient stands upright.

As will be more apparent below, the annuloplasty ring 30 has a relatively complex shape for which a reference plane is difficult to define, as it is for the mitral annulus. However, for purposes of comparing the annuloplasty ring 30 of the present invention against those that preceded it, or those that might be constructed after, it is fair to say that most heart surgeons and annuloplasty ring designers recognize that early planar rings were intended to be implanted in the nominal "annular plane," (with the understanding that anatomical differences exist from patient to patient). From there, some rings were modified to have an upward bow in the anterior side to conform to the systolic anatomical contour of the anterior aspect of most mitral annuluses. These days, physicians, designers and manufacturers understand the term upward bow when describing mitral annuloplasty rings to mean upward from the original planar design, or reference plane. Therefore, if a mitral annuloplasty ring is described in product literature as having an upward bow, the understanding is that the ring bows upward at that location from adjacent segments that define the reference plane. Likewise, if the literature describes two bows with one relatively higher than the other, it is understood to mean their respective heights parallel to the flow axis, or perpendicular to the reference plane. Otherwise, some rings can be placed on a flat surface and by dint of gravity settle into any number of orientations.

Another way to look at the reference orientation of the ring 30 is that two points on opposite sides of the ring 30, typically along or near the major axis 34, are at the lowest points of the saddle shape and reside in the "reference plane." For example, as seen in Figs. 3-5, points 40 and 42 may be thought of as lying in a reference plane that is perpendicular to the flow axis 32 of the ring (and parallel to the line of view of Figs. 3-5). The ring connects points 40, 42 to one another as seen in the upper and lower portions of Fig. 6 to form a closed ring. The ring 30 is intended to be implanted at the mitral annulus with points 40 and 42 approximately adjacent the commissures of the valve. So, relative to Fig. 6, the surgeon will implant the ring such that the upper segment of the ring 30 will register with the anterior aspect or leaflet of the mitral annulus, and the lower segment will register with the posterior aspect or leaflet.

A still further way to define the flow axis or reference plane is to note the orientation that the ring assumes relative to a delivery holder. Annuloplasty rings are conventionally mounted on holders that include a central hub for connecting to a handle, and the central hub defines an axis. Thus, to see the relative heights of the sides of a ring it may be placed so that the central hub of its holder is perpendicular to a reference surface.

With reference again to Fig. 6, a pair of trigone markers **T₁** and **T₂** are shown on the ring 30 corresponding to the approximate location of the fibrous commissures or trigones of the mitral annulus when the ring is implanted. An anterior segment **AS** extends around the upper portion of the ring 30 between the trigone markers **T₁**, **T₂**. When the ring 30 is implanted, the anterior segment **AS** will coincide with the anterior aspect of the mitral annulus. The anterior segment **AS** is upwardly curved or bowed to better conform to the anterior aspect of the native annulus, as will be shown below in Figs. 7-10 with respect to an exemplary ring body.

The remainder of the ring 30 aside from the anterior segment **AS** between the trigone markers **T₁**, **T₂** will be termed the posterior portion, shown divided into three sequential segments labeled **P₁**, **P₂**, and **P₃** (counter-clockwise from the first trigone marker **T₁**). The precise angular dividing line between these three segments is not standardized, though they are intended to generally correspond to and abut the three visible cusps of the posterior leaflet of the mitral valve. In an exemplary embodiment, the three segments are approximately equal in angular dimension, and the middle segment **P₂** is symmetric about the minor axis 36. In an alternative embodiment, the ring 30 is asymmetric so as to bulge outward more toward **P₁** or **P₃**, such as seen in U.S. Patent Publication No. 2005/0131533, filed June 30, 2004, and entitled Annuloplasty Rings for Repair of Abnormal Mitral Valves,

It should be noted that annuloplasty rings are shaped and marked (e.g., with the trigone markers **T₁** and **T₂**) so as to orientation-specific, such that the anterior segment is intended to be implanted against the anterior aspect of the mitral annulus, and vice versa with respect to the posterior portion. Annuloplasly rings such as those of the present invention are not rotatable within the annulus. That is, a particular orientation is indicated on the packaging, or by reference to the trigone markers **T₁**, **T₂**, and the ring is constructed to be specific to that orientation. A surgeon would not, for instance, implant the ring with the anterior segment AS adjacent the posterior aspect of the mitral annulus.

The exemplary annuloplasty ring 30 has a rounded isosceles triangular shape in plan view because of an outward bulge 50 centered in the middle segment **P₂** of the posterior portion of the ring. Stated another way, the middle segment **P₂** or the posterior portion of the ring has an outward curve (convexity) in comparison with the adjacent segments **P₁** and **P₃** which are relatively straight. The exemplary annuloplasty ring 30 bulges outward on the posterior side relative to a conventional 3:4 ratio "D-shaped" annuloplasty ring, such as the relaxed shape of a Carpentier-Edwards Physio® annuloplasty ring available from Edwards Lifesciences of Irvine, CA (www.edwards.com). The divergence from the "D-shape" essentially commences mid-way along each of the side segments **P₁** and **P₃** and spans an angle which desirably is between 90-130°, and more preferably about 128°.

As mentioned above, the outward bulge 50 preferably result in a minor-major axis dimensional ratio of 3.5:4 (87.5%), although the present invention encompasses rings having an outward bulge 50 that produces ratios of between about 3.3:4 (82.5%) and 4:4 (100%).

It is important to note that although the minor axis dimension 38a increases relative to conventional D-shaped rings, the major axis dimension 38b will remain substantially the same. Furthermore, although the outward bulge 50 is shown within the middle segment **P₂** of the posterior portion of the ring, the entire posterior portion below the major axis 34 may be affected. That is, the outward bulge 50 may extend into one or both of the first and third segments **P₁** and **P₃** of the posterior portion. In that case, the overall triangular shape would expand outward so as to be more "D-shaped."

In conjunction with the outward bulge 50, the annuloplasty ring 30 also includes an upward bow 60 in the posterior portion, as seen in Figs. 3 and 5. The magnitude **hₚ** of the posterior upward bow 60 is indicated in Fig. 5 and is desirably reduced from previous rings to between about 2.9-4.5 mm. The upward bow 60 may or may not be formed in the ring 30 around the same angular extent as the outward bulge 50. In a preferred embodiment, both the outward bulge 50 and upward bow 60 are centered along the minor axis 36, although one or both may be asymmetrically offset as mentioned above.

The annuloplasty ring 30 further exhibits an upward bow 62 on the anterior segment **AS**. The magnitude of this bow 62 is given as **hₚ** in Fig. 5, and is desirably greater than the magnitude **hₚ** of the posterior upward bow 60. In an exemplary form, the anterior bow 62 is at least 1 mm and preferably between 1-2 mm higher than the posterior bow 60.

As mentioned, and as conventional, the annuloplasty ring 30 construction includes an inner generally rigid core and an outer suture-permeable covering. Fig. 3A shows a cross-section of the exemplary ring 30 consisting of an inner ring body 70 surrounded by an elastomeric interface 72 and an outer fabric sleeve 74. The elastomeric interface 72 may be silicone rubber molded around the ring body 70, or a similar expedient. The elastomeric interface 72 provides bulk to the ring for ease of handling and implant, and permits passage of sutures though not significantly adding to the anchoring function of the outer fabric sleeve 74. The fabric sleeve 74 may be any biocompatible material such as Dacron® (polyethylene terepthalate). Desirably, the elastomeric interface 72 and fabric sleeve 74 is thicker along the outside of the ring 30 than on the inside to provide a bulked platform through which to pass sutures. An alternative interface is simply wrapped, bunched or rolled fabric, with no silicone.

With reference now to Figs. 7-10, an exemplary mitral annuloplasty ring body 70 is shown in several illustrative views. These views omit the silicone sleeve and fabric covering. It should be understood that the shape of the completed ring 30 as seen in Figs. 2-6 follows closely the shape of the inner ring body 70 which provides its primary structural support.

The annuloplasty ring body 70 has a rounded isosceles triangular shape in plan view and is oriented about a central flow axis 82. The flow axis 82 defines an upward direction and a downward direction, corresponding to the top and bottom of the page relative to the ring body 70 as seen in Fig. 8. Looking along the flow axis 82 in Fig. 7, the ring body 70 has a major axis 84 perpendicular to a minor axis 86, the major and minor axes being orthogonal to the flow axis 82. A minor axis dimension 88a extends vertically across the interior of the ring body 70 in plan view. Likewise, a major axis dimension 88b extends horizontally across the interior of the ring body. Desirably, the ratio of the minor axis dimension 88a to the major axis dimension 88b is about 3.5:4 (87.5%), although the present invention provides a ring having an outward bulge 90 that may create a minor axis/major axis ratio of between about 3.3:4 (82.5%) and 4:4 (100%).

The annuloplasty ring body 70 has a rounded isosceles triangular shape in plan view with a relatively straight anterior segment **AS** extends around the upper portion of the ring body 70 in Fig. 7 between trigones **T₁** and **T₂** (the trigones are typically not marked on the ring body but are indicated here for clarity). From the anterior segment AS, two relatively straight side segments 94a, 94b converge on the posterior portion of the ring and terminate in the convex outward posterior bulge 90. The combined extent of the side segments 94a, 94b and posterior bulge 90 corresponds to the posterior segments **P₁**, **P₂**, and **P₃** of the ring 30. The side segments 94a, 94b join to the anterior segment **AS** at rounded anterior corners 95a, 95b.

The outline of an isosceles triangle is seen with its sides coinciding with an inner edge of the three relatively straight sides of the ring body 70. The triangle has a long side along the relatively straight anterior segment **AS** and two equal slightly shorter sides along the side segments 94a, 94b. Two equal anterior corner angles α supplement a posterior vertex angle β. Desirably, the ring body 70 has a shape in which 50° > α > 60°, and 60° > β > 80°. In a preferred shape, 55° > α > 56°, and 68° > β > 70°.

The extent of the outward bulge 90 of the ring body 70 has been discussed above in general terms. However, it is important to understand that the major and minor axes can be measured at different points on the ring body 70, and with respect to the completed ring 30 as seen in Figs. 2-6. The completed ring 30 includes the inner ring body 70, a surrounding sleeve or band of silicone, and a fabric covering therearound. Therefore, the major and minor axes can be measured to the inside or outside dimensions of the ring body, and likewise with respect to the completed ring. As an analysis of just one of these measurements, the following table indicates the actual values of the major and minor axes as measured across the interior of the ring body 70 (dimensions 88b and 88a, respectively, in Fig. 7) for eight different exemplary rings, and also gives percentage ratios of the minor axis to the major axis as measured across the inner ring body 70.

| Ring size (mm) | Major axis (mm) | Minor Axis (mm) | Across ring body 70 ID |
|---|---|---|---|
| 26 | 26.0 | 22.8 | 87.5% |
| 28 | 28.0 | 24.5 | 87.5% |
| 30 | 30.0 | 26.2 | 87.5% |
| 32 | 32.0 | 28.0 | 87.5% |
| 34 | 34.0 | 29.7 | 87.5% |
| 36 | 36.0 | 31.5 | 87.5% |
| 38 | 38.0 | 33.2 | 87.4% |
| 40 | 40.0 | 35.0 | 87.5% |

The ratio of the minor axis to the major axis desirably falls within a range of 3.3:4 to 4:4 (82.5%-100%), more preferably between about 3.44:4 to 3.6:4 (86%-90%), and most preferably 3.5:4 (87.5%). It should be understood that the dimensional parameters set forth herein with respect to the ring body 70 correspond closely with those of the completed ring 30, though the outward bulk of the outer suture-permeable covering seen in Fig. 3A will cause the outer diameter to be proportionally larger in the assembled ring.

The exemplary annuloplasty ring body 70 bulges outward on the posterior side relative to a conventional 3:4 ratio "D-shaped" annuloplasty ring body, such as the relaxed shape of a Carpentier-Edwards Physio® annuloplasty ring available from Edwards Lifesciences of Irvine, CA (www.edwards.com). A dashed outline 96 of such a conventional 3:4 ratio "D-shaped" annuloplasty ring body is shown to illustrate the more pronounced outward bulge 90 of the present ring body 70.

It is important to note that although the minor axis dimension 88a increases relative to conventional D-shaped ring bodies (i.e., dashed outline 96), the major axis dimension 88b will remain substantially the same (for a given ring size). Furthermore, although the outward bulge 90 is shown centered within the middle of the posterior portion of the ring, the entire posterior portion below the major axis 84 may be affected. That is, the outward bulge 90 may extend into one or both of the side segments 94a, 94b of the posterior portion. Preferably, the divergence from the "D-shape" essentially commences mid-way along each of the side segments 94a, 94b and spans an angle λ which desirably is between 90-130°, and more preferably about 128°.

In the illustrated embodiment, the ring body 70 exhibits two opposed lower flats 100a, 100b located in the side segments 94a, 94b that, as seen in Fig. 8, define the lowermost portions of the ring body. If the ring body 70 is placed on a flat base surface, the flats 100a, 100b rest on the base surface while the anterior segment AS and posterior bulge 90 rise upward from the base surface. The flats 100a, 100b lie in a reference plane 101 (Fig. 8A) of the ring that essentially corresponds to earlier planar rings. That is, the anterior segment **AS** and posterior bulge 90 rise upward from the reference plane, or from the lowermost portions of the ring body. The flats 100a, 100b are believed to provide enhanced support for the lateral aspects of the mitral annulus in the **P₁** and **P₃** regions.

The exemplary ring body 70 of the present invention includes an upward bow 102 in the posterior portion, as seen in Figs. 8 and 8A. The magnitude **hₚ** of the posterior upward bow 102 is indicated in Fig. 8A and is desirably between about 2.9-4.5 mm. Another way to express the magnitude of the upward bow 102 is as a percentage of the major axis dimension 88b, as the upward bow generally increases with increasing ring size. In this context, the posterior upward bow 102 is desirably between about 9%-13%, and more preferably about 11%, of the major axis dimension 88b.

The upward bow 102 may or may not be formed in the ring body 70 around the same angular extent as the outward bulge 90. In a preferred embodiment, both the outward bulge 90 and upward bow 102 are centered along the minor axis 86, although one or both may be asymmetrically offset as mentioned above. The upward bow 102 may span a peripheral extent of the ring body 70 that is larger than the outward bulge 90. For example, the upward bow 102 may be centered about the minor axis 86 and commence at symmetric locations on both sides of the posterior portion mid-way along each of the side segments 94a, 94b, as indicated in Fig. 7, so as to span an angle λ which desirably is between 90-130°, and more preferably about 128°. Alternatively, the upward bow 102 may be asymmetric about the minor axis 86 and extend farther around the ring into the side segments 94a, 94b of the posterior portion. The shape may be customized to match a perceived corrective need.

As seen in Figs. 8 and 8A, the anterior segment **AS** is also upwardly curved or bowed at 104 to better conform to the anterior aspect of the native annulus in systole. The magnitude **hₐ** of the anterior bow 104 is indicated in Fig. 8A, and desirably ranges between about 4-7 mm relative to the adjacent sides 94a, 94b. Again, another way to express the magnitude of the magnitude **hₐ** of the anterior bow 104 is as a percentage of the major axis dimension 88b. In this context, the magnitude **hₐ** is desirably between about 14%-20%, and more preferably about 16%, of the major axis dimension 88b. This upward curvature has been adopted in certain more rigid rings of the prior art to more faithfully conform to the upward contour at the anterior aspect of the mitral annulus, which is pronounced during systole. It should be noted that the height **hₐ** of the anterior bow 104 is greater than previous anterior bows, such as in the Carpentier-Edwards Physio® annuloplasty ring available from Edwards Lifesciences of Irvine, CA.

An important aspect of the present invention is the existence of an enhanced anterior bow in combination with a somewhat reduced posterior how. This is distinct from an earlier ring designed to correct similar pathologies disclosed in co-pending U.S. Patent Publication No. 2006/0129236, filed February 2, 2006, and entitled Annuloplasty Ring for Mitral Valve Prolapse,
The earlier ring disclosed a posterior bow, and all anterior side that could be planar or bowed. None of the embodiments described an enhanced anterior bow 104 as discussed above, or an anterior bow that rises higher than a posterior bow 102. It is believed that in some cases a reduced posterior bow is necessary to avoid dehiscence, the likelihood of which tends to increase with higher posterior bows. At the same time, in some cases an enhanced anterior bow is believed to more faithfully match the systolic anatomy of the certain mitral annuluses than previous rings with anterior bows.

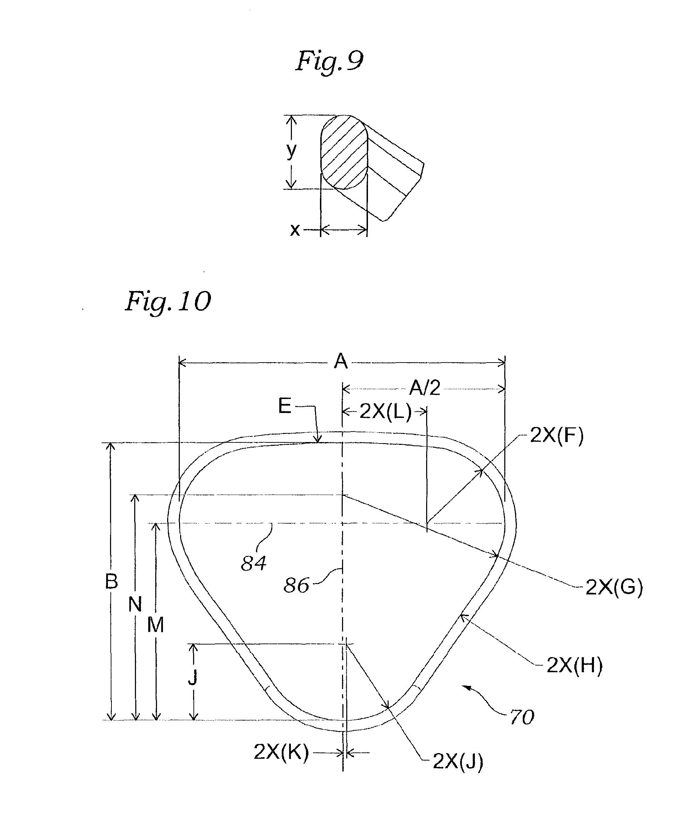

The present invention contemplates an annuloplasty ring with an upward and outward posterior bow 102, and an enhanced anterior bow 104, but also with particular dimensions, ratios, and contours to optimize performance. One such dimensional configuration is the cross-sectional shape. Fig. 9 illustrates a radial cross-section through the ring body 70, which is preferably constant throughout. As shown, the cross-section has a vertically-oriented oval configuration with a radial dimension **x** and a greater axial dimension **y**. This configuration is desirable as it provides sufficient strength (as required by a stress analysis) but minimizes the radial thickness (**x** dimension) so that a relatively robust sewing band around the periphery may be utilized without compromising orifice area. A thicker sewing band as seen in Fig. 3A facilitates implant.

The particular shape shown, a vertically-oriented oval, is believed most desirable because of its rounded contours and consequent lack of stress risers. In a preferred embodiment, the cross-section of the ring body 70 is constant around its periphery, although a varying cross-section to vary the flexibility around the ring body is also contemplated. In one exemplary embodiment, the radial dimension **x** is between about 60% to 70% of the axial dimension **y**. Additionally, the top and bottom rounded ends of the cross-section are desirably complete semi-circles having a diameter that equals the radial dimension **x**. For example, **x** = 0.049 inches (1.24 mm) and y = 0.079 inches (2.00 mm).

As mentioned above, the illustrated ring body 70 may be constructed of a single, homogenous length of a relatively rigid material such as titanium. As such, the ring body 70 will substantially resist distortion when subjected to the stress imparted thereon by the mitral valve annulus of an operating human heart. It should be understood that less rigid materials may provide some radial bending flexibility for the ring to accommodate in and out movement of the annulus during systolic-diastolic cycles. For instance, a polymer ring formed with the cross-section shown in Fig. 9 may flex radially, though it will be stiffer axially. Or, the ring body 70 may be formed from a plurality of concentric radially thin bands of a more rigid material such as titanium, which reduces radial rigidity without compromising axial strength. Desirably, the ring body 70 possesses a higher vertical area moment of inertia than its horizontal area moment of inertia, thus resulting in greater bending flexibility about the flow axis (i.e., in and out flexibility) than about radial axes (i.e., up and down flexibility). This construction helps preserves the integrity of the upward bows 102, 104, which is important to correct the pathology that led to mitral valve prolapse. In all cases, however, the ring materials are elastic and will substantially resist distortion of the preferred (or manufactured) ring shape when subjected to annulus forces.

Figs. 10 illustrate further preferred dimensions and contours for the exemplary ring body 70. As is common, annuloplasty rings are sized in even 2 mm increments, and various dimensional parameters indicated by letters (A, B, etc.) are generally proportional for rings between about 24 and 40 mm. However, the cross-section of each of these differently sized rings desirably remains the same, as discussed above with respect to Fig. 9, and do not become proportionally larger with larger rings, though a set of rings with proportionally sized cross-sections is entirely feasible.

The reader should also note the particular contours of the ring body periphery as seen in the plan view of Fig. 10, and also with reference to the features shown in Fig. 7. Beginning at the top, the anterior aspect **AS** has a very slight outward curvature (convexity) indicated by the radius E. The anterior corners 95a, 95b of the ring body near the trigones **T₁** and **T₂** (see Fig. 7) exhibit a curvature having a radius F that is centered about a point on the major axis 84 a distance L from the minor axis 86, approximately half way to the ring body 70. The extent of the corner curvature (radius F) is approximately 90°. The corners 95a, 95b terminate near the plane of the major axis 84, which plane is desirably spaced a distance M from the posterior apex. M is approximately 71 % of the interior dimension B of the minor axis 86.

Small convex segments having a radius G (centered on the minor axis 86 a distance N from the posterior apex) that have a lesser curvature than the upper corners lead to the relatively straight side segments 94a, 94b (see Fig. 7) having a very slight inward (concave) curvature with a radius H. The side segments 94a, 94b define the beginning of the divergence from a conventional D-shape, and make up a majority of the first and third segments P₁ and P₃ of the posterior portion (see Fig. 6). The segments 94a, 94b are illustrated as slightly concave, though they may be straight or slightly convex as well. These sections essentially narrow the ring body 70 on the posterior side so that the outward bulge 90 is isolated in the middle or P₂ segment, rather than affecting the adjacent P₁ or P₃ segments. That is, rather than comprising a smooth, relatively gradual change of curvature, the outward bulge 90 is formed by a segment centered in the posterior portion that has a smaller radius of curvature than the adjacent sections.

Finally, the outermost posterior apex straddling the minor axis 86 has a radius of J that is desirably about the same as the radius F of the anterior corners 95a, 95b. It should be noted that the centers of curvature of the two sides of the outermost posterior apex are slightly offset from the minor axis 86, as indicated, which slightly widens or flattens the posterior point of the ring body relative to a constant curvature. This outermost curved portion spans an angular or peripheral extent of between about 80-90° within the total outward divergence (angle λ, Fig. 7) of about 128°.

Fig. 8 best illustrates the particular contours of the upward bows 102, 104. It should be noted that the upward bow 102 has gradual segments adjacent the side segments 94a, 94b and then a more dramatic mid-segment. As mentioned above, the entire angular extent of the upward bow 102 is approximately is between 90-130° symmetric about the minor axis 86. The more dramatic mid-segment spans an angular extent approximately 80-90°, and in this respect corresponds approximately to the preferred peripheral span of the outward radial bow 90.

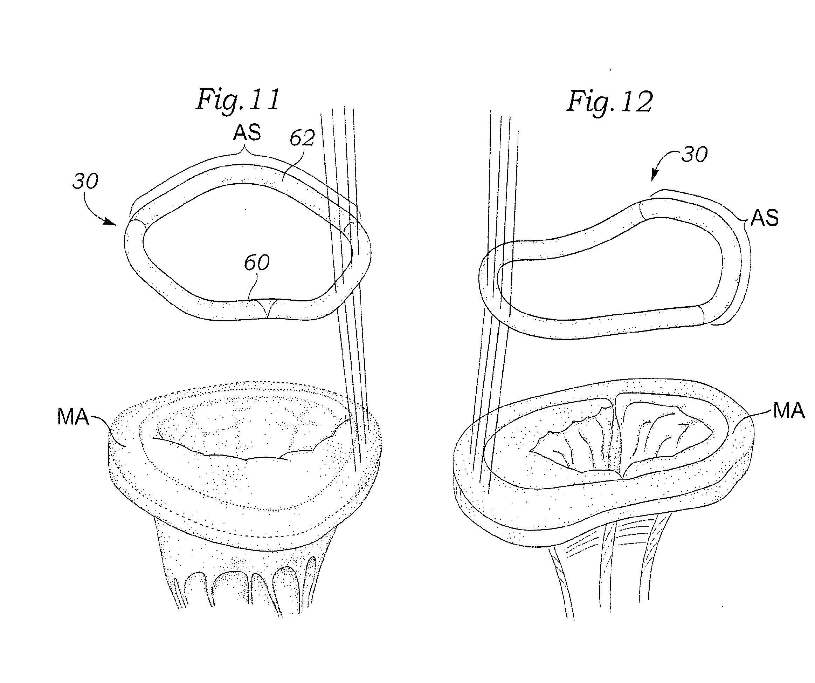

Figs. 11 and 12 illustrate the exemplary annuloplasty ring 30 in perspective above a mitral annulus MA that is misshapen with one or more leaflets thickened and lengthened from their normal configurations. A typical delivery technique is to pass ring 30 down an array of pre-anchored sutures (only 2 looped sutures shown for clarity) in a so-called parachute delivery. In this way, the annulus conforms to the ring 30 when they are secured together.

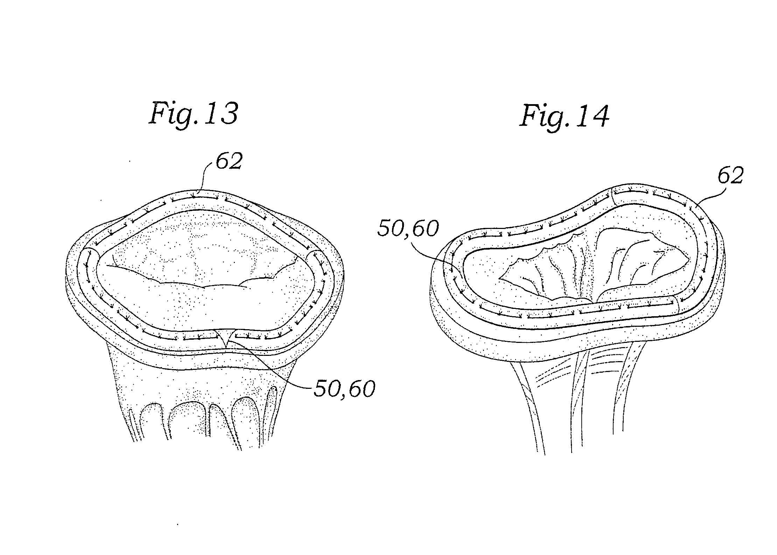

Figs. 13 and 14 illustrate the implantation of the annuloplasty ring 30 in the mitral annulus to correct the condition of Figs. 11 and 12. The suture loops are tied off using conventional means. The outward and upward posterior bow 50, 60 of the ring 30 is shown located at the posterior aspect of the annulus. The shape of the ring 30 on the posterior side lifts the posterior aspect of the mitral annulus, and helps reduce the "slack" existent in the anterior leaflet. Also, the enhanced anterior bow 62 better conforms to the annulus in systole. As a result, the leaflets properly coapt to substantially eliminate regurgitation.

Although not shown, the ring 30 is usually advanced through the body on a holder, ultimately passing through the left atrium. Many holders are available in the art, though the most preferred are disclosed in co-pending U.S. Application Serial No. *, filed concurrently herewith, and entitled "Apparatus, System, and Method for Delivering an Annuloplasty Ring,"

The annuloplasty ring of the present invention is believed to more effectively correct the pathology seen with some cases of mitral valve prolapse because it accommodates the longer and/or thicker leaflets instead of attempting to perform a sliding annuloplasty, which is sometimes perceived as more surgical art than an exact science. The combination of the outward bulge and the upward bow on the posterior side of the ring is believed to provide rigid support tor the posterior leaflet from which it can more effectively coapt with the anterior leaflet. The annuloplasty ring essentially "pulls" the posterior leaflet outward and upward which reduces its slack or floppiness. Also, in some cases the enhanced anterior bow more correctly conforms to the anterior aspect in systole, which helps shape the anterior leaflet for proper coaptation. Furthermore, the ring should pull the coaptation point outward and upward and away from the LVOT. This should reduce the incidence of SAM and LVOT obstruction and mitral regurgitation post-repair.

While the invention has been described in its preferred embodiments, it is to be understood that the words which have been used are words of description and not of limitation. Therefore, changes may be made within the appended claims without departing from the true scope of the invention.

## Claims

1. A mitral annuloplasty ring (30) comprising:
a closed and generally rigid ring body arranged around a flow axis (32) having an upward direction and a downward direction, the downward direction corresponding to the direction of blood flow through the mitral valve annulus when the annuloplasty ring is implanted, the ring body having at least two lowermost points (40, 42) on opposite sides of the ring body that lie in a reference plane generally perpendicular to the flow axis (32);
the ring body having, in top plan view along the flow axis (32), a rounded isosceles triangular configuration with a long relatively straight anterior segment (AS) substantially extending between two trigones (T₁, T₂) and intended to be implanted against the anterior aspect of the mitral annulus, and two shorter relatively straight posterior segments joined (P₁, P₃) at a convex posterior apex (P₂) and together defining a posterior portion of the ring body, the posterior portion (P₁, P₂, P₃) intended to be implanted against the posterior aspect of the mitral annulus; and
wherein, in elevational view parallel to the reference plane, each of the anterior segment (AS) and the posterior portion (P₁, P₂, P₃) has an upward bow therein relative to the two lowermost points (40, 42) of the ring (30), **characterised in that** the anterior segment (AS) bows upward farther from the reference plane than the posterior portion (P₁, P₂, P₃).

2. The mitral annuloplasty ring of claim 1, wherein the ring body defines a minor axis (36) extending between and bisecting the anterior segment (AS) and posterior portion (P₁, P₂, P₃) and a major axis (34) extending perpendicularly thereto, the major and minor axes (34, 36) being generally perpendicular to the flow axis (32) and each having dimensions across the ring body, and wherein the posterior portion (P₁, P₂, P₃) of the ring body has an outward bulge (50) more pronounced than adjacent sections.

3. The mitral annuloplasty ring of claim 2, wherein the upward bow (60) in the posterior portion (P₁. P₂, P₃) has an angular extent approximately equal to the outward bulge (50).

4. The mitral annuloplasty ring of claim 1, wherein the magnitude of the posterior upward bow (60) is between about 2.9-4.5mm.

5. The mitral annuloplasty ring of claim 1, wherein the ring body defines a minor axis (36) extending between and bisecting the anterior segment (AS) and posterior portion (P₁, P₂, P₃) and a major axis (34) extending perpendicularly thereto, the major and minor axes (34, 36) being generally perpendicular to the flow axis (32) and each having dimensions across the ring body, and wherein the relative magnitude of the posterior upward bow (60) is between about 9%-13% of the major axis dimension (38b).

6. The mitral annuloplasty ring of claims 1 or 5, wherein the ring body defines a minor axis (36) extending between and bisecting the anterior segment (AS) and posterior portion (P₁, P₂, P₃) and a major axis (34) extending perpendicularly thereto, the major and minor axis (34, 36) being generally perpendicular to the flow axis (32) and each having dimensions across the ring body, and wherein the relative magnitude of the anterior upward bow (62) is between about 14%-20% of the major axis dimension (38b).

7. A mitral annuloplasty ring comprising:
a closed and generally rigid ring body (70) arranged around a flow axis (82) having an upward direction and a downward direction, the downward direction corresponding to the direction of blood flow through the mitral valve annulus when the annuloplasty ring (30) is implanted, the ring body (70) having at least two lowermost points (30) on opposite sides of the ring body (70) that lie in a reference plane generally perpendicular to the flow axis (82);
the ring body (70) having, in top plan view along the flow axis (82), a relatively straight anterior segment (AS) substantially extending between two trigones (T₁, T₂)and intended to be implanted against the anterior aspect of the mitral annulus, and a generally convex posterior portion (P₁, P₂, P₃) of the ring body extending between two trigones (T₁, T₂) opposite the anterior segment (AS) and intended to be implanted against the posterior aspect of the mitral annulus, and with a minor axis (86) extending between the anterior segment (AS) and an apex of the posterior portion (P₁, P₂, P₃) and a major axis (84) extending perpendicularly thereto across the widest part of the ring body (70), the major and minor axes (84, 86) being generally perpendicular to the flow axis (82) and each having dimensions (88a, b)across the ring body, and wherein the posterior portion (P₁, P₂, P₃) of the ring body (70) has an outward bulge (90) that creates a minor axis to major axis dimension ratio of between about 3.3:4 (82.5%) and 4:4 (100%); and
wherein, in elevational view parallel to the reference plane, each of the anterior segment (AS) and the posterior portion (P₁, P₂, P₃) has an upward bow therein relative to the two lowermost points of the ring, **characterised in that** the anterior segment (AS) bows upward farther from the reference plane than the posterior portion (P₁, P₂, P₃).

8. The mitral annuloplasty ring of claim 7, wherein the ring body (70) has, in top plan view along the flow axis (82), a rounded isosceles triangular configuration defined by the anterior segment (AS) and two shorter relatively straight posterior segments (P₁, P₃) joined at a convex posterior apex (P₂) and together defining the posterior portion.(P₁, P₂, P₃).

9. The mitral annuloplasty ring of claim 7, wherein the magnitude of the posterior upward bow (90) is between about 2.9-4.5mm, and

10. The mitral annuloplasy ring of claim 7, wherein the relative magnitude of the posterior upward bow (90) is between about 9%-13%of the major axis dimension(88b).

11. The mitral annuloplasty ring of claim 10, wherein the relative magnitude of the anterior upward bow AS is between about 14%-20% of the major axis dimension (88b).

12. The mitral annuloplasty ring of claim 7, wherein the magnitude of the anterior upward bow AS is between about 4-7mm.

13. A mitral annuloplasty ring comprising:
a closed and generally rigid ring body (70) arranged around a flow axis (82) having an upward direction and a downward direction, the downward direction corresponding to the direction of blood flow through the mitral valve annulus when the annuloplasty ring (30) is implanted, the ring body (70) having at least two lowermost points on opposite sides of the ring body (70) that lie in a reference plane generally perpendicular to the flow axis (82);
the ring body having, in top plan view along the flow axis (82), a relatively straight anterior segment (AS) substantially extending between two trigones (T₁, T₂) and intended to be implanted against the anterior aspect of the mitral annulus, and two posterior segments (P₁, P₃) joined at a convex posterior apex (P₂,) and together defining a posterior portion (P₁, P₂, P₃) of the ring body, the posterior portion (P₁, P₂, P₃) intended to be implanted against the posterior aspect of the mitral annulus; and
wherein, in elevational view parallel to the reference plane, each of the anterior segment (AS) and the posterior portion (P₁. P₂, P₃) has an upward bow therein relative to the two lowermost points of the ring (30), **characterised in that** the anterior segment (AS) bows upward farther from the reference plane than the posterior portion.(P₁. P₂, P₃).

14. The mitral annuloplasty ring of claim 13, wherein the ring body (70) defines lowermost flat segments (100a, 100b) on opposite sides of the posterior portion (P_{1,} P₂, P₃) in which are located the two lowermost points of the ring.

15. The mitral annuloplasty ring of claim 13, wherein the upward bow in the anterior segment (AS) is between 1-2mm higher than the upward bow in the posterior portion (P₁, P₂, P₃).

16. The mitral annuloplasty ring of claim 13, wherein the magnitude of the posterior upward bow (90) is between about 2.9-4.5mm.

17. The mitral annuloplasty ring of claim 13, wherein the magnitude of the anterior upward bow (AS) is between about 4-7mm.

18. The mitral annuloplasty ring of claim 13, wherein the ring body defines a minor axis (86) extending between and bisecting the anterior segment (AS) and posterior portion (P_{1,} P₂, P₃) and a major axis (84) extending perpendicularly thereto, the major and minor axes (84, 86) being generally perpendicular to the flow axis (82) and each having dimensions across the ring body, and wherein the relative magnitude of the posterior upward bow (90) is between about 9%-13% of the major axis dimension (88b).

19. The mitral annuloplasty ring of claim 13, wherein the ring body (70) defines a minor axis (86) extending between and bisecting the anterior segment (AS) and posterior portion (P₁, P₂, P₃) and a major axis (84) extending perpendicularly thereto, the major and minor axes (84, 86) being generally perpendicular to the flow axis (82) and each having dimensions across the ring body, and wherein the relative magnitude of the anterior upward bow (AS) is between about 14%-20% of the major axis dimension (88b).

## Patentansprüche

1. Mitral-Anuloplastiering (30) umfassend:
einen geschlossenen und im Allgemeinen starren Ringkörper, der um eine Strömungsachse (32), die eine Aufwärtsrichtung und eine Abwärtsrichtung aufweist, angeordnet ist, wobei die Abwärtsrichtung der Richtung der Blutströmung durch den Mitralklappenring entspricht, wenn der Anuloplastiering implantiert ist, wobei der Ringkörper wenigstens zwei unterste Punkte (40, 42) an gegenüberliegenden Seiten des Ringkörpers aufweist, die in einer Referenzebene im Allgemeinen senkrecht zur Strömungsachse (32) liegen;
wobei der Ringkörper in einer Draufsicht entlang der Strömungsachse (32) eine abgerundete gleichschenklige Dreieckskonfiguration aufweist, mit einem langen, relativ geraden vorderen Segment (AS), das sich im Wesentlichen zwischen zwei Trigoni (T₁, T₂) erstreckt und dafür vorgesehen ist, gegen die vordere Seite des Mitralrings implantiert zu werden, und zwei kürzeren, relativ geraden hinteren Segmenten (P₁, P₃), die an einem konvexen hinteren Scheitelpunkt (P₂) verbunden sind und gemeinsam einen hinteren Teil des Ringkörpers definieren, wobei der hintere Teil (P₁, P₂, P₃) dafür vorgesehen ist, gegen die hintere Seite des Mitralrings implantiert zu werden; und
wobei in einer Seitenansicht parallel zu der Referenzebene das vordere Segment (AS) und der hintere Teil (P₁, P₂, P₃) jeweils in sich einen Bogen nach oben relativ zu den zwei untersten Punkten (40, 42) des Rings (30) aufweisen, **dadurch gekennzeichnet, dass** das vordere Segment (AS) sich nach oben weiter von der Referenzebene weg biegt als der hintere Teil (P₁, P₂, P₃).

2. Mitral-Anuloplastiering nach Anspruch 1, wobei der Ringkörper eine Nebenachse (36), die sich zwischen dem vorderen Segments (AS) und dem hinteren Teil (P₁, P₂, P₃) erstreckt und diese halbiert, und eine Hauptachse (34), die sich senkrecht hierzu erstreckt, definiert, wobei die Haupt- und die Nebenachse (34, 36) im Allgemeinen senkrecht zu der Strömungsachse (32) sind und jeweils Ausdehnungen quer über den Ringkörper aufweisen, und wobei der hintere Teil (P₁, P₂, P₃) des Ringkörpers eine Ausbuchtung (50) nach Außen aufweist, die ausgeprägter ist als benachbarte Abschnitte.

3. Mitral-Anuloplastiering nach Anspruch 2, wobei der Aufwärtsbogen (60) im hinteren Teil (P₁, P₂, P₃) eine Winkelausdehnung ungefähr gleich der Ausbuchtung (50) nach Außen aufweist.

4. Mitral-Anuloplastiering nach Anspruch 3, wobei die Größe des hinteren Aufwärtsbogens (60) zwischen etwa 2,9 und 4,5 mm beträgt.

5. Mitral-Anuloplastiering nach Anspruch 1, wobei der Ringkörper eine Nebenachse (36), die sich zwischen dem vorderen Segment (AS) und dem hinteren Teil (P₁, P₂, P₃) erstreckt und diese halbiert, und eine Hauptachse (34), die sich senkrecht hierzu erstreckt, definiert, wobei die Haupt- und die Nebenachse (34, 36) im Allgemeinen senkrecht zu der Strömungsachse (32) sind und jeweils Ausdehnungen quer über den Ringkörper aufweisen, und wobei die relative Größe des hinteren Aufwärtsbogens (60) zwischen etwa 9 % und 13 % der Hauptachsenausdehnung (38b) beträgt.

6. Mitral-Anuloplastiering nach einem der Ansprüche 1 oder 5, wobei der Ringkörper eine Nebenachse (36), die sich zwischen dem vorderen Segment (AS) und dem hinteren Teil (P₁, P₂, P₃) erstreckt und diese halbiert, und eine Hauptachse (34), die sich senkrecht hierzu erstreckt, definiert, wobei die Haupt- und die Nebenachse (34, 36) im Allgemeinen senkrecht zu der Strömungsachse (32) sind und jeweils Ausdehnungen quer über den Ringkörper aufweisen, und wobei die relative Größe des vorderen Aufwärtsbogens (62) zwischen etwa 14 % und 20 % der Hauptachsenausdehnung (38b) beträgt.

7. Mitral-Anuloplastiering (30) umfassend:
einen geschlossenen und im Allgemeinen starren Ringkörper (70), der um eine Strömungsachse (82), die eine Aufwärtsrichtung und eine Abwärtsrichtung aufweist, angeordnet ist, wobei die Abwärtsrichtung der Richtung der Blutströmung durch den Mitralklappenring entspricht, wenn der Anuloplastiering (30) implantiert ist, wobei der Ringkörper (70) wenigstens zwei unterste Punkte (30) an gegenüberliegenden Seiten des Ringkörpers (70) aufweist, die in einer Referenzebene im Allgemeinen senkrecht zur Strömungsachse (82) liegen;
wobei der Ringkörper in einer Draufsicht entlang der Strömungsachse (82) ein langes, relativ gerades vorderes Segment (AS), das sich im Wesentlichen zwischen zwei Trigoni (T₁, T₂) erstreckt und dafür vorgesehen ist, gegen die vordere Seite des Mitralrings implantiert zu werden, und einen im Allgemeinen konvexen hinteren Teil (P₁, P₂, P₃) des Ringkörpers, der sich zwischen zwei Trigoni (T₁, T₂) gegenüberliegend dem vorderen Segment (AS) erstreckt und dafür vorgesehen ist, gegen die hintere Seite des Mitralrings implantiert zu werden, aufweist, mit einer Nebenachse (86), die sich zwischen dem vorderen Segment (AS) und einem Scheitelpunkt des hinteren Teils (P₁, P₂, P₃) erstreckt, und einer Hauptachse (84), die sich senkrecht hierzu quer über den größten Teil des Ringkörpers (70) erstreckt, wobei die Haupt- und die Nebenachse (84, 86) im Allgemeinen senkrecht zur Strömungsachse (82) sind und jeweils Ausdehnungen (88a, b) quer über den Ringkörper aufweisen, und wobei der hintere Teil (P₁, P₂, P₃) des Ringkörpers (70) eine Ausbuchtung (90) nach Außen aufweist, die ein Ausdehnungsverhältnis der Nebenachse zu der Hauptachse zwischen etwa 3,3:4 (82,5 %) und 4:4 (100 %) erzeugt; und
wobei in einer Seitenansicht parallel zu der Referenzebene das vordere Segment (AS) und der hintere Teil (P₁, P₂, P₃) jeweils in sich einen Bogen nach oben relativ zu den zwei untersten Punkten des Rings aufweisen, **dadurch gekennzeichnet, dass** das vordere Segment (AS) sich nach oben weiter von der Referenzebene weg biegt als der hintere Teil (P₁, P₂, P₃).

8. Mitral-Anuloplastiering nach Anspruch 7, wobei der Ringkörper (70) in einer Draufsicht entlang der Strömungsachse (82) eine abgerundete gleichschenklige Dreieckskonfiguration aufweist, die durch das vordere Segment (AS) und zwei kürzere, relativ gerade hintere Segmente (P₁, P₃), die an einem konvexen hinteren Scheitelpunkt (P₂) verbunden sind und gemeinsam den hinteren Teil (P₁, P₂, P₃) definieren, definiert ist.

9. Mitral-Anuloplastiering nach Anspruch 7, wobei die Größe des hinteren Aufwärtsbogens (90) zwischen etwa 2,9 und 4,5 mm beträgt.

10. Mitral-Anuloplastiering nach Anspruch 7, wobei die relative Größe des hinteren Aufwärtsbogens (90) zwischen etwa 9 % bis 13 % der Hauptachsenausdehnung (88b) beträgt.

11. Mitral-Anuloplastiering nach Anspruch 10, wobei die relative Größe des vorderen Aufwärtsbogens AS zwischen etwa 14 % und 20 % der Hauptachsenausdehnung (88b) beträgt.

12. Mitral-Anuloplastiering nach Anspruch 7, wobei die Größe des vorderen Aufwärtsbogens AS zwischen etwa 4 und 7 mm beträgt.

13. Mitral-Anuloplastiering (30) umfassend:
einen geschlossenen und im Allgemeinen starren Ringkörper (70), der um eine Strömungsachse (82), die eine Aufwärtsrichtung und eine Abwärtsrichtung aufweist, angeordnet ist, wobei die Abwärtsrichtung der Richtung der Blutströmung durch den Mitralklappenring entspricht, wenn der Anuloplastiering (30) implantiert ist, wobei der Ringkörper (70) wenigstens zwei unterste Punkte an gegenüberliegenden Seiten des Ringkörpers (70) aufweist, die in einer Referenzebene im Allgemeinen senkrecht zur Strömungsachse (82) liegen;
wobei der Ringkörper in einer Draufsicht entlang der Strömungsachse (82) ein langes, relativ gerades vorderes Segment (AS), das sich im Wesentlichen zwischen zwei Trigoni (T₁, T₂) erstreckt und dafür vorgesehen ist, gegen die vordere Seite des Mitralrings implantiert zu werden, und zwei hintere Segmente (P₁, P₃), die an einem konvexen hinteren Scheitelpunkt (P2) verbunden sind und gemeinsam einen hinteren Abschnitt (P₁, P₂, P₃) des Ringkörpers definieren, aufweist, wobei der hintere Teil (P₁, P₂, P₃) dafür vorgesehen ist, gegen die hintere Seite des Mitralrings implantiert zu werden; und
wobei in einer Seitenansicht parallel zu der Referenzebene das vordere Segment (AS) und der hintere Teil (P₁, P₂, P₃) jeweils in sich einen Bogen nach oben relativ zu den zwei untersten Punkten des Rings (30) aufweisen, **dadurch gekennzeichnet, dass** das vordere Segment (AS) sich nach oben weiter von der Referenzebene weg biegt als der hintere Teil (P₁, P₂, P₃).

14. Mitral-Anuloplastiering nach Anspruch 13, wobei der Ringkörper (70) unterste flache Segmente (100a, 100b) an gegenüberliegenden Seiten des hinteren Teils (P₁, P₂, P₃) definiert, in denen die zwei untersten Punkte des Rings angeordnet sind.

15. Mitral-Anuloplastiering nach Anspruch 13, wobei der Aufwärtsbogen im vorderen Segment (AS) zwischen 1 und 2 mm höher ist als der Aufwärtsbogen im hinteren Teil (P₁, P₂, P₃).

16. Mitral-Anuloplastiering nach Anspruch 13, wobei die Größe des hinteren Aufwärtsbogens (90) zwischen etwa 2,9 und 4,5 mm beträgt.

17. Mitral-Anuloplastiering nach Anspruch 13, wobei die Größe des vorderen Aufwärtsbogens (AS) zwischen etwa 4 und 7 mm beträgt.

18. Mitral-Anuloplastiering nach Anspruch 13, wobei der Ringkörper eine Nebenachse (86), die sich zwischen dem vorderen Segment (AS) und dem hinteren Teil (P₁, P₂, P₃) erstreckt und diese halbiert, und eine Hauptachse (84), die sich senkrecht hierzu erstreckt, definiert, wobei die Haupt- und die Nebenachse (84, 86) im Allgemeinen senkrecht zu der Strömungsachse (82) sind und jeweils Ausdehnungen quer über den Ringkörper aufweisen, und wobei die relative Größe des hinteren Aufwärtsbogens (90) zwischen etwa 9 % und 13 % der Hauptachsenausdehnung (88b) beträgt.

19. Mitral-Anuloplastiering nach Anspruch 13, wobei der Ringkörper (70) eine Nebenachse (86), die sich zwischen dem vorderen Segment (AS) und dem hinteren Teil (P₁, P₂, P₃) erstreckt und diese halbiert, und eine Hauptachse (84), die sich senkrecht hierzu erstreckt, definiert, wobei die Haupt- und die Nebenachse (84, 86) im Allgemeinen senkrecht zu der Strömungsachse (82) sind und jeweils Ausdehnungen quer über den Ringkörper aufweisen, und wobei die relative Größe des vorderen Aufwärtsbogens (AS) zwischen etwa 14 % und 20 % der Hauptachsenausdehnung (88b) beträgt.

## Revendications

1. Anneau (30) d'annuloplastie comprenant :
un corps annulaire, fermé et d'une manière générale rigide, disposé autour d'un axe (32) d'écoulement ayant une direction vers le haut et une direction vers le bas, la direction vers le bas correspondant à la direction du courant de sang dans l'anulus de la valvule mitrale lorsque l'anneau d'annuloplastie est implanté, le corps annulaire ayant au moins deux points (40, 42) les plus bas sur des côtés opposés du corps annulaire qui se trouvent dans un plan de référence, d'une manière générale perpendiculaire à l'axe (32) d'écoulement ;
le corps annulaire ayant, en vue en plan par le haut le long de l'axe d'écoulement, une configuration arrondie en triangle isocèle ayant un segment (AS) antérieur long relativement droit, s'étendant sensiblement entre deux trigones (T₁, T₂) et destiné à être implanté sur la face antérieure de l'anulus mitral et deux segments postérieurs plus courts relativement droits, réunis (P₁, P₃) en un sommet (P₂) postérieur convexe et définissant ensemble une partie postérieure du corps annulaire, la partie (P₁, P₂, P₃) postérieure destinée à être implantée sur la face postérieure de l'anulus mitral ; et
dans lequel, en vue en élévation parallèlement au plan de référence, chacun du segment (AS) antérieur et de la partie (P₁, P₂, P₃) postérieure a en son sein une courbe allant vers le haut par rapport aux deux points (40, 42) les plus bas de l'anneau (30), **caractérisé en ce que** le segment (AS) antérieur se courbe vers le haut plus loin du plan de référence que la partie (P₁, P₂, P₃) postérieure.

2. Anneau d'annuloplastie mitrale suivant la revendication 1, dans lequel le corps annulaire définit un petit axe (36) s'étendant entre le segment (AS) antérieur et la partie (P₁, P₂, P₃) postérieure et les bissectant et un grand axe (34) s'y étendant perpendiculairement, les grand et petit axes (34, 36) étant d'une manière générale perpendiculaires l'axe (32) d'écoulement chacun des dimensions à travers le corps annulaire, dans lequel la partie (P₁, P₂, P₃) postérieure du corps annulaire a un renflement (50) vers l'extérieur plus prononcé que des sections voisines.

3. Anneau d'annuloplastie mitrale suivant la revendication 2, dans lequel la courbe (60) allant vers le haut dans la partie (P₁, P₂, P₃) postérieure a une étendue angulaire à peu près égale au renflement (50) allant vers l'extérieur.

4. Anneau d'annuloplastie mitrale suivant la revendication 1, dans lequel l'amplitude de la courbe (60) postérieure allant vers le haut est comprise entre environ 2,9 et 4,5 mm.

5. Anneau d'annuloplastie mitrale suivant la revendication 1, dans lequel le corps annulaire définit un petit axe (36) s'étendant entre le segment (AS) antérieur et la partie (P₁, P₂, P₃) postérieure et les bissectant et un grand axe (34) s'y étendant perpendiculairement, le grand et le petit axes (34, 36) étant d'une manière générale perpendiculaires à l'axe (32) d'écoulement et ayant chacun des dimensions à travers le corps annulaire, et dans lequel l'amplitude relative de la courbe (60) postérieure allant vers le haut représente environ de 9% à 13% de la dimension (38b) du grand axe.

6. Anneau d'annuloplastie mitrale suivant la revendication 1 ou 5, dans lequel le corps annulaire définit un petit axe (36) s'étendant entre le segment (AS) antérieur et la partie (P₁, P₂, P₃) postérieure et les bissectant et un grand axe (34) s'y étendant perpendiculairement, le grand et le petit axes (34, 36) étant d'une manière générale perpendiculaires à l'axe (32) d'écoulement et ayant chacun des dimensions à travers le corps annulaire, et dans lequel l'amplitude relative de la courbe (60) antérieure allant vers le haut représente environ de 14% à 20% de la dimension (38b) du grand axe.

7. Anneau d'annuloplastie mitrale comprenant :
un corps (70) annulaire fermé et d'une manière générale rigide, disposé autour d'un axe (82) d'écoulement ayant une direction vers le haut et une direction vers le bas, la direction vers le bas correspondant à la direction d'écoulement du sang dans l'anulus de la valvule mitrale, lorsque l'anneau (30) d'annuloplastie est implanté, le corps (70) annulaire ayant au moins deux points (30) les plus bas sur des côtés opposés du corps (70) annulaire, qui se trouvent dans un plan de référence d'une manière générale perpendiculaire à l'axe (82) d'écoulement ;
le corps (70) annulaire ayant, en vue en plan par le haut le long de l'axe (82) d'écoulement, un segment (AS) antérieur relativement droit, s'étendant sensiblement entre deux trigones (T₁, T₂) et destiné à être implanté sur la face antérieure de l'anulus mitrale et une partie (P₁, P₂, P₃) postérieure généralement convexe du corps annulaire, s'étendant entre deux trigones (T₁, T₂) opposés au segment (AS) antérieur et destinée à être implantée sur la face postérieure de l'anulus mitral, et ayant un petit axe (86) s'étendant entre le segment (AS) antérieur et un sommet de la partie (P₁, P₂, P₃) postérieure et un grand axe (84) s'y étendant perpendiculairement à travers la partie la plus large du corps (70) annulaire, le grand et le petit axes (84, 86) étant d'une manière générale perpendiculaires à l'axe (82) d'écoulement et ayant chacun des dimensions (88a, b) à travers le corps annulaire, et dans lequel la partie (P1, P2, P3) postérieure du corps (70) annulaire a un renflement (90) vers l'extérieur, qui crée un rapport de dimension du petit axe au grand axe compris entre environ 3,3:4 (82,5%) et 4:4 (100%) ; et
dans lequel, en vue en élévation parallèlement au plan de référence, chacun du segment (AS) antérieur et de la partie (P₁, P₂, P₃) postérieure a en son sein une courbe allant vers le haut par rapport aux deux points les plus bas de l'anneau, **caractérisé en ce que** le segment (AS) antérieur se courbe vers le haut plus loin du plan de référence que la partie (P₁, P₂, P₃) postérieure.

8. Anneau d'annuloplastie mitrale suivant la revendication 7, dans lequel le corps (70) annulaire a, dans une vue en plan par le haut le long de l'axe (82) d'écoulement, une configuration arrondie en triangle isocèle définie par le segment (AS) antérieur et par deux segments (P₁, P₃) postérieurs droits relativement plus courts, réunis en un sommet (P₂) postérieur convexe et définissant ensemble la partie (P₁, P₂, P₃) postérieure.

9. Anneau d'annuloplastie mitrale suivant la revendication 7, dans lequel l'amplitude de la courbe (90) postérieure allant vers le haut est comprise entre 2,9 et 4,5 mm, et

10. Anneau d'annuloplastie mitrale suivant la revendication 7, dans lequel l'amplitude relative de la courbe (90) postérieure allant vers le haut représente environ de 9% à 13% de la dimension (88b) du grand axe.

11. Anneau d'annuloplastie mitrale suivant la revendication 11, dans lequel l'amplitude relative de la courbe AS antérieure allant vers le haut représente environ de 14% à 20% de la dimension (88b) du grand axe.

12. Anneau d'annuloplastie mitrale suivant la revendication 7, dans lequel l'amplitude de la courbe AS antérieure allant vers le haut est comprise entre 4 et 7 mm.

13. Anneau d'annuloplastie mitrale comprenant :
un corps (70) annulaire, fermé et d'une manière générale rigide, disposé autour d'un axe (82) d'écoulement ayant une direction vers le haut et une direction vers le bas, la direction vers le bas correspondant à la direction d'écoulement du sang dans l'anulus de la valvule mitrale, lorsque l'anneau (30) d'annuloplastie est implanté, le corps (70) annuaire ayant au moins deux points (30) les plus bas sur des côtés opposés du corps (70) annulaire, qui se trouvent dans un plan de référence d'une manière générale perpendiculaire à l'axe (82) d'écoulement ;
le corps annulaire ayant, en vue en plan par le haut le long de l'axe (82) d'écoulement, un segment (AS) antérieur relativement droit, s'étendant sensiblement entre deux trigones (T1, T2) et destiné à être implanté sur la face antérieure de l'anulus mitral et deux segments (P₁, P₃) postérieurs réunis en un sommet (P₂) postérieur convexe et définissant ensemble une partie (P₁, P₂, P₃) postérieure du corps annulaire, la partie (P₁, P₂, P₃) antérieure destinée à être implantée sur la face postérieure de l'anulus mitral ; et
dans lequel, en vue en élévation parallèlement au plan de référence, chacun des segments (AS) antérieurs et de la partie (P₁, P₂, P₃) postérieure a en son sein une courbe allant vers le haut par rapport aux deux points les plus bas de l'anneau (30), **caractérisé en ce que** le segment (AS) antérieur se courbe vers le haut plus loin du plan de référence que la partie (P₁, P₂, P₃) postérieure.

14. Anneau d'annuloplastie mitrale suivant la revendication 13, dans lequel le corps (70) annulaire définit des segments (100a, 100b) plats les plus bas sur des côtés opposés de la partie (P1, P2, P3) postérieure, dans lesquels sont disposés les deux points les plus bas de l'anneau.

15. Anneau d'annuloplastie mitrale suivant la revendication 13, dans lequel la courbe allant vers le haut dans le segment (AS) antérieur est plus haute entre 1 à 2 mm que la courbe allant vers le haut dans la partie (P₁, P₂, P₃) postérieure.

16. Anneau d'annuloplastie mitrale suivant la revendication 13, dans lequel l'amplitude de la courbe (90) postérieure allant vers haut est comprise entre environ 2,9 et 4,5 mm.

17. Anneau d'annuloplastie mitrale suivant la revendication 13, dans lequel l'amplitude de la courbe (AS) antérieure allant vers haut est comprise entre environ 4 et 7 mm.

18. Anneau d'annuloplastie mitrale suivant la revendication 13, dans lequel le corps annulaire définit un petit axe (86) s'étendant entre le segment (AS) antérieur et la partie (P₁, P₂, P₃) postérieure et les bissectant et un grand axe (84) s'y étendant perpendiculairement, le grand et le petit axes (84, 86) étant d'une manière générale perpendiculaires à l'axe (82) d'écoulement et ayant chacun des dimensions à travers le corps annulaire, et dans lequel l'amplitude relative de la courbe (90) postérieure allant vers le haut représente environ de 9% à 13% de la dimension (88b) du grand axe.

19. Anneau d'annuloplastie mitrale suivant la revendication 13, dans lequel le corps (70) annulaire définit un petit axe (86) s'étendant entre le segment (AS) antérieure et la partie (P₁, P₂, P₃) postérieure et les bissectant et un grand axe (84) s'y étendant perpendiculairement, le grand et le petit axes (84, 86) étant d'une manière générale perpendiculaires à l'axe (82) d'écoulement et ayant chacun des dimensions à travers le corps annulaire, et dans lequel l'amplitude relative de la courbe (AS) postérieure allant vers le haut représente environ de 14% à 20% de la dimension (88b) du grand axe.
